Europäisches Patentamt

European Patent Office

Office européen des brevets

(11) Publication number: **0 406 436 A1**

# EUROPEAN PATENT APPLICATION
## published in accordance with Art. 158(3) EPC

(21) Application number: 89913133.8

(22) Date of filing: 22.11.89

(86) International application number:
PCT/JP89/01187

(87) International publication number:
WO 90/05784 (31.05.90 90/12)

(51) Int. Cl.5: **C12P 19/32**, C12N 15/54,
C12N 15/70, C12N 1/21,
//C12R1:19,C12R1:425,
C12R1:13

(30) Priority: 22.11.88 JP 2956/87

(43) Date of publication of application:
09.01.91 Bulletin 91/02

(84) Designated Contracting States:
AT BE CH DE ES FR GB IT LI LU NL

(71) Applicant: KYOWA HAKKO KOGYO CO., LTD.
6-1, Ohtemachi 1-chome
Chiyoda-ku Tokyo 100(JP)

(72) Inventor: FUJIO, Tatsuro
6-29-1, Sagamidai Sagamihara-shi
Kanagawa 228(JP)
Inventor: IIDA, Akihiro
4-17-9, Morino Machida-shi
Tokyo 194(JP)

(74) Representative: Vossius & Partnerner
Siebertstrasse 4 P.O. Box 86 07 67
D-8000 München 86(DE)

(54) **PROCESS FOR PREPARING 5'-INOSINIC ACID.**

(57) The invention relates to process for preparing 5'-inosinic acid (IMP) from a carbon source, hypoxanthine (Hyp), and a phosphoric acid donating material using a microorganism as an enzyme source. In this process, use is made of both a microorganism having the activity of yielding 5-phosphoribosyl-l-pyrophosphoric acid (PRPP) or its precursor from a carbon source and another microorganism having the activity of yielding IMP from both of Hyp or its precursor and PRPP or its precursor.

Fig. 1

Specification

Process for Producing 5'-Inosinic Acid

Technical Field

This invention relates to a process for producing 5'-inosinic acid (hereinafter referred to as "IMP"). IMP has a taste-enhancing effect and is widely used as a seasoning agent.

Background Art

So far known processes for producing IMP include (1) a process by enzymatically decomposing ribonucleic acid extracted from yeast cells and deaminating the resulting 5'-adenylic acid ["Kakusan Hakko" compiled by Amino Acid-Nucleic Acid Shudankai, p.59 (1976)], (2) a process by chemically phosphorylating inosine produced by fermentation ["Kakusan Hakko", compiled by Amino Acid - Nucleic Acid Shudankai, p.209 (1976)], (3) a process for the production by direct fermentation using a mutant strain belonging to the genus Brevibacterium or Corynebacterium (Japanese Published Examined Patent Application No. 26640/75), etc.

It is known that IMP is biosynthesized from hypoxanthine (hereinafter referred to as "Hyp") and 5-phosphoribosyl-1-pyrophosphate (hereinafter referred to as "PRPP") by utilizing the salvage synthesis system. It is known that hypoxanthine phosphoribosyl-transferase (EC 2.4. 2.8) is involved in the reaction and that IMP is formed in the culture medium by culturing, in a medium containing Hyp, a microorganism having the enzymatic activity ["Kakusan Hakko" compiled by Amino Acid - Nucleic Acid Shudankai, p.35 (1976)].

It has been so far known that IMP is formed from Hyp and PRPP, and that its yield is quite low. No process has been available for producing IMP at a low cost in an industrial scale. This is because supply of PRPP as a necessary substrate for the reaction to form IMP from Hyp is considered to be insufficient. However, PRPP is an expensive, unstable reagent and thus it is not economically advantageous to add PRPP as a substrate. Accordingly, development of a process for producing IMP more efficiently at a low cost in an industrial scale has been desired.

Disclosure of Invention

The present inventor has made various investigations in the development of a process for producing IMP at a low cost in an industrial scale. Consequently it has been found that IMP can be produced more efficiently from Hyp and a carbon source by using a microorganism having an activity to form PRPP or its precursor from the carbon source, and using a microorganism having an activity to form IMP from Hyp and PRPP together in the reaction to form IMP from Hyp and PRPP. Thus the present invention has been established.

The present invention provides a process for producing IMP, which comprises carrying out IMP formation reaction in the presence of a microorganism having an activity to form PRPP or its precursor, a microorganism having an activity to form IMP from Hyp or it precursor and PRPP or its precursor (hereinafter sometimes referred to as "converting microorganism"), or their cultures or treated products of their cells; Hyp or its precursor or a material containing these substances, a carbon source; and a phosphate donor, thereby forming and accumulating IMP in the reaction solution, and recovering IMP from the reaction solution.

Furthermore, the present invention provides a recombinant DNA comprising a DNA fragment containing a gene coding for an enzyme having an activity to phosphoribosylate Hyp and a vector DNA, and microorganisms carrying the recombinant DNA. By using such microorganisms as the converting microorganisms, IMP can be produced in much higher yield from Hyp and PRPP.

The present invention will be described in detail below.

As a microorganism having an activity to form PRPP or its precursor (hereinafter referred to as "a microorganism having an activity to form PRPP"), any microorganism can be used, so long as it has such an activity. The microorganism is specifically exemplified by:

Brevibacterium ammoniagenes ATCC 6871
Brevibacterium ammoniagenes ATCC 6872
Brevibacterium ammoniagenes ATCC 21062
Brevibacterium ammoniagenes ATCC 21295
Brevibacterium ammoniagenes ATCC 21477
Brevibacterium ammoniagenes ATCC 21478
Brevibacterium ammoniagenes ATCC 21479
Brevibacterium ammoniagenes ATCC 21480
Arthrobacter citreus            ATCC 11624
Corynebacterium glutamicum      ATCC 13032
Bacillus subtilis               ATCC 14618
Escherichia coli                ATCC 33525
Escherichia coli                ATCC 11303
Staphylococcus aureus           ATCC 4012
Saccharomyces sake              ATCC 20018
Candida zeilanoides             ATCC 20356
Torulopsis psychrophila         ATCC 22163, etc.

By culturing these strains according to the ordinary culturing procedure, cultures or cells of microorganisms having an activity to form PRPP or its precursor can be obtained. That is, these strains are cultured in an ordinary medium containing an appropriate carbon source, a nitrogen source, inorganic materials, amino acids, vitamins, etc. under aerobic conditions while adjusting the temperature, pH, etc.

Culturing is carried out under aerobic conditions, for example, by shaking culture, aeration-stirring culture, etc. at a temperature of 20 to 40°C, preferably 25 to 35°C, usually for 10 to 120 hours, while maintaining pH around neutral.

As a microorganism (converting microorganism) having an activity to form IMP from Hyp and PRPP or its precursor, any microorganism can be used, so long as it has such an activity. The microorganism is specifically exemplified by:

| Escherichia coli | ATCC 10798 |
| Escherichia coli | ATCC 11303 |
| Bacillus subtilis | ATCC 14617 |
| Flavobacterium devorans | ATCC 10829 |
| Serratia marcescens | FERM BP-1291 |
| Salmonella typhimurium | ATCC 19585 |
| Lactobacillus casei | ATCC 7469 |
| Brevibacterium ammoniagenes | ATCC 6872 |
| Corynebacterium glutamicum | ATCC 13032, etc. |

Furthermore, strains endowed with an activity to phosphoribosylate Hyp constructed by a molecular breeding procedure such as cell fusion with these microorganisms, etc. are suitable for the purpose.

IMP can be more efficiently formed by use of a microorganism having an improved activity to form IMP from Hyp and PRPP or its precursor, prepared by introducing, into a microorganism, a recombinant DNA which is constructed by isolating a DNA fragment containing a gene coding for an enzyme having an activity to phosphoribosylate Hyp from the above-mentioned microorganism and inserting the DNA fragment into a vector DNA.

As a gene coding for an enzyme having an activity to phosphoribosylate Hyp, any gene can be used, so long as it codes for an enzyme having such an activity. For example, genes coding for hypoxanthine phosphoribosyl transferase (EC 2.4.2.8, hereinafter referred to as "HPRTase"), guanine·xanthine·phosphoribosyl transferase (EC2.4.2.22, hereinafter referred to as "GPRTase"), etc. are suitable for the purpose. As donors of the genes coding for HPRTase or GPRTase, any microorganism can be used, so long as it is a microorganism having an activity to form IMP from Hyp and PRPP, as mentioned above. Specifically, a gene coding for HPRTase or GPRTase originating from Serratia marcescens (Example 1 or 2) and a gene coding for GPRTase of Escherichia coli (Example 3) can be enumerated. By inserting a DNA fragment containing the genes into a plasmid vector and transforming a strain with the resulting recombinant DNA, the strain which overexpresses with HPRTase and GPRTase can be obtained.

By culturing these microorganisms according to the ordinary culturing procedure, cultures, cells or their treated products having an activity to form IMP from Hyp and PRPP can be obtained. That is, these microorganisms are cultured in an ordinary medium containing a suitable carbon source, a nitrogen source, inorganic materials, amino acids, vitamins, etc. under aerobic conditions, while adjusting the temperature, pH, etc.

Culturing is carried out under aerobic conditions, for example, by shaking culture, aeration-stirring culture, etc. Culturing temperature is 20 to 50°C, preferably 28 to 40°C. It is desirable to maintain the pH of the medium around neutral during the culturing. Culture period is usually 1 to 48 hours.

As a carbon source for culturing a strain having an activity to form PRPP and a strain having an activity to form IMP from Hyp and PRPP, carbohydrates and sugar alcohols such as glucose, fructose, sucrose, maltose, mannitol, sorbitol, etc.; various alcohols such as glycerol and organic acids such as pyruvic acid, lactic acid, citric acid, etc.; and various amino acids such as glutamic acid, methionine, lysine, etc. can be used. Furthermore, such natural nutrient sources such as starch hydrolyzate, molasses, blackstrap molasses, white rice bran, cassava, bagasse, corn steep liquor, etc. can be used, so long as they can be assimilated by strains to be used.

As a nitrogen source, ammonia; various inorganic and organic ammonium salts such as ammonium chloride, ammonium sulfate, ammonium carbonate, ammonium acetate, etc.; amino acids such as glutamic acid, glutamine, methionine, etc.; and nitrogen-containing organic materials such as peptones, NZ-amine, corn steep liquor, meat extract, yeast extract, casein hydrolyzate, fish meal or its digested products, etc. can be used.

As inorganic materials, potassium dihydrogen phosphate, sodium monohydrogen phosphate, magnesium sulfate, sodium chloride, calcium chloride, iron chloride, copper sulfate, manganese chloride, ammonium molybdate, zinc sulfate, etc. are added to the medium, when required. Vitamins, amino acids, minerals, nucleic acids, etc. necessary for the growth of the microorganisms are added to the medium, when required.

IMP can be obtained by contacting Hyp or its precursor or a material containing these substances, a carbon source, a phosphate donor and other necessary components with thus obtained culture of a micoorganism having an activity to form PRPP, or its cells or a liquor containing their treated products, and thus obtained culture of a converting microorganism or its cells or a liquor containing their treated products. A PRPP precursor may be added thereto, when required.

Contacting of a culture or cells of a microorganism having an activity to form PRPP or its precursor or their treated products, a culture or cells of a converting microorganism having an activity to form IMP from PRPP with Hyp or their products, a carbon source, a phosphate donor, etc. can be carried out by (1) culturing the microorganisms separately and mixing the microorganisms together after the completion of culturing, (2) by mixing Hyp or its precursor and a culture or cells of the converting microorganism or their treated products with a microorganism having an activity to form PRPP at any time in the course from the starting point of culturing the microorganism having an activity to form PRPP to the end point of the culturing, (3) by adding Hyp or its precursor and a culture or cells of a microorganism having an activity to form PRPP or their treated products to a converting microorganism at any time in the course from the starting point of culturing the converting microorganism to the end point of the culturing, or (4) by mix-culturing a microorganism having an activity to form PRPP and a converting microorganism and adding Hyp and its precursor to the mix-culture at any desired time.

As an Hyp precursor, inosine can be exemplified, but any other compound can be used, so long as it can be microbially or enzymatically easily changed to Hyp and can be changed to Hyp by any of strains of the microorganisms having an activity to form PRPP and converting microorganisms or by the activity possessed by these strains. The precursor can be used after changing it to Hyp by a pretreatment such as an enzymatic treatment, etc., or while conducting changing the precursor to Hyp by addition of an enzyme, etc. in parallel with the conversion reaction. Furthermore, purified product and roughly purified product of inosine, inosine fermentation liquor, a supernatant liquor free from the cells and its concentrate can be used, so long as they do not inhibit the reaction to phosphoribosylate Hyp to IMP.

As substrates for forming PRPP, a carbon source and a phosphate donor are used. Any carbon source is used so long as it can be utilized by a microorganism having an activity to form PRPP to be used. As the carbon source, any of carbohydrates such as glucose, ribose, arabinose, lactose, maltose, sucrose, mannitol, sorbitol, trehalose, molasses, blackstrap molasses, other glucide, starch hydrolyzate, etc.; organic acids such as pyruvic acid, lactic acid, acetic acid, α-ketoglutaric acid, etc.; and amino acids such as glycine, alanine, aspartic acid, glutamic acid, glutamine, etc. can be used. Phosphate compounds such as acetyl phosphate, carbamyl phosphate, creatine phosphate, etc. can also be used.

As the phosphate donor, any of sodium salts, potassium salts, magnesium salts, etc. of inorganic phosphoric acid such as orthophosphoric acid, pyrophosphoric acid, polyphosphoric acid, polymetaphosphoric acid, etc. can be used. Organic phosphate compounds such as acetyl phosphate, carbamyl phosphate, creatine phosphate, fructose-1,6-diphosphate, etc. can also be used. It is desirable to keep its concentration within a range of 10 to 400 mM.

As a PRPP precursor, any of substances produced by a microorganism capable of producing PRPP or its precursor and readily changed to PRPP by a converting microorganism can be used. For example, any of adenosine 5'-triphosphate (hereinafter referred to as "ATP"), ATP-related compounds such as adenosine 5'-diphosphate, adenosine 5'-monophosphate, adenosine and adenine, pentoses or their phosphates such as ribose, ribose 5-phosphate, ribose 1-phosphate, etc., can be used, so long as they can promote formation of PRPP without inhibition of phosphoribosylation reaction of Hyp and PRPP to IMP. If a sufficient amount of PRPP can be biosynthesized from the carbon source, its addition is not particularly necessary.

When a microorganism having a recombinant DNA comprising a DNA fragment containing a gene coding for an enzyme having an activity to phosphoribosylate Hyp and a vector DNA is used as a converting microorganism, a PRPP supply source is not particularly limited, and the PRPP can be added as a reagent without using a microorganism having an activity to form PRPP. However, IMP can be formed in much higher yield by using a microorganism having an activity to form PRPP.

The phosphoribosylating reaction of Hyp or its precursor to IMP is carried out by adding, if required, magnesium ions, a surfactant and/or an organic solvent to the above-mentioned mixed liquor while adjusting a pH to 6-10, preferably 7-8 and keeping the mixed liquor at 20 - 50°C for 1-48 hours. It is desirable that an Hyp concentration is in a range of 1 to 100 mg/ml during the phosphoribosylating reaction of Hyp to IMP.

As the treated products of cultures or cells of each of a microorganism having an activity to form PRPP and a converting microorganism, concentrates and dried products of cultures, cells obtained by centrifuging the cultures, frozen cells, dried products, freeze-dried products, acetone-treated products, surfactant and/or organic solvent-treated products and lytic enzyme-treated products, of cells, immobilized cells, etc. can be enumerated. Furthermore, liquors containing PRPP biosynthetic enzyme or phosphoribosylating enzyme, extracted from the cells and purified preparates, immobilized products, etc., of these enzymes can also be used.

As a surfactant, any of cationic surfactants such as polyoxyethylene-stearylamine (for example, Nymeen S-215, made by Nippon Oil and Fats Co., Ltd., hereinafter surfactants made by Nippon Oil and Fats Co., Ltd., were hereinafter used unless otherwise specified), cetyltrimethyl-ammonium bromide, Cation FB, Cation F2-40E, etc.; anionic surfactants such as sodium oleylamide sulfate, Newlex TAB, Rapisol 80, etc.; ampholytic surfactants such as polyoxyethylenesorbitan, monostearate (for example, Nonion ST221), etc.; and tertiary amine PB, hexadecyldimethylamine, etc. can be used, so long as it can promote biosynthesis of PRPP and/or phosphoribosylating reaction of Hyp and PRPP to IMP. The surfactant is used at a concentration of usually 0.1 to 50 mg/ml, preferably 1 to 20 mg/ml.

As an organic solvent, toluene, xylene, aliphatic alcohols, benzene, ethyl acetate, etc. can be used at a concentration of 0.1 to 50 $\mu$l/ml, preferably 1 to 20 $\mu$l/ml. It is desirable to keep the concentration of magnesium ions in a range of 4 to 400 mM during the biosynthetic reaction of PRPP and the phosphoribosylating reaction of. Hyp and PRPP to IMP. When the amount of magnesium ions to be brought into the reaction system from the culture or cells falls within the above-mentioned concentration range, it is not necessary to add magnesium ions to the reaction system. When the amount is not enough on the other hand, magnesium ions must be added thereto, so as to make the concentration within the above-mentioned range. In case that the magnesium ions are in excess, the reaction system must be diluted. As·magnesium ions, any of inorganic or organic salts of magnesium can be used. After the end of reaction, IMP formed and accumulated in the reaction solution can be recovered according to the ordinary procedure using ion exchange resin, etc.

Brief Description of Drawing

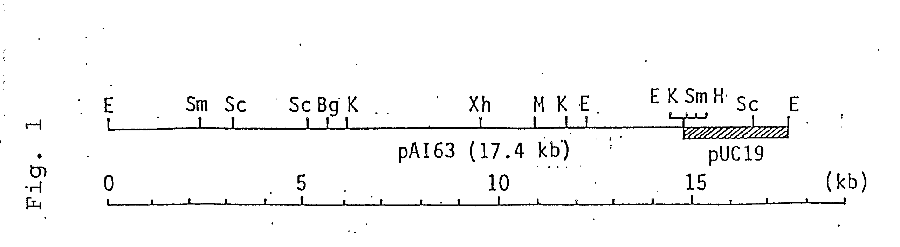

Fig. 1 is a restriction enzyme-cleavage map of plasmid pAI63, where E shows EcoRI, Sm shows SmaI, Sc shows ScaI, Bg shows BglII, K shows KpnI, Xh shows XhoI, M shows MluI and H shows HindIII.

Examples of the present invention are given below.

Example 1

Construction of a recombinant plasmid containing a DNA fragment containing an HPRTase gene originating from Serratia marcescens.

(1) Construction of a recipient strain for cloning HPRTase gene:

Escherichia coli Sø 609 strain [B. Jochimsen, P. Nygaad, and T. Vestergard: Mol. Gen. Genet., 143, 85 (1975), available from the E. coli Genetic Stock Center of Yale University], which was deficient in HPRTase and GPRTase and requires inosine and/or Hyp, was subjected to a mutation treatment by N-methyl-N'-nitro-N-nitrosoguanidine (hereinafter referred to as NTG) to thereby obtain an AI675 strain released from the host restriction as the recipient used in obtaining an HPRTase gene (sometimes hereinafter referred to as "hpt gene").

The induction of mutation by NTG was carried out in the following manner. That is, a culture obtained by reciprocal shaking culture (190 rpm) at 37°C in a large-scale test tube containing 10 mℓ of LB medium overnight was centrifuged at 3,000 rpm for 15 minutes to recover cells. The precipitated cells were washed twice with physiological saline solution (an aqueous 0.9% sodium chloride solution), and then suspended in the physiological saline solution so as to make a concentration of $5 \times 10^8$ cells per mℓ. NTG was added to the suspension to make a final concentration of 500 μg/mℓ and the suspension was allowed to stand at room temperature for 45 minutes. After washing twice with the physiological saline solution, NTG-treated cells were obtained.

Detection of the presence or absence of the host restriction was carried out by culturing the mutation-treated strain on an LB liquid medium comprising 10 g/l bactotrypton (made by Difco), 5 g/l yeast extract (made by Difco) and 5 g/l sodium chloride, adjusted to pH 7.2, at 30°C for 20 hours and then carrying out cross-streak by use of the cultured microorganism on an LB plate medium (the LB liquid medium further containing 1.5% agar made by Difco) together with a λ phage prepared from Escherichia coli C600 (r⁻m⁻) strain (ATCC33525) according to the well known procedure [J. H. Miller: Experiments in Molecular Genetics, Cold Spring Harbor Lab. (1972)] on the basis of the sensitivity of λ phage to the microorganism as an indication. The genotype of AI675 is ara, rpsL, deoD, hsdR, purH/J, Δpro-gpt-lac, thi and hpt.

(2)   Shotgun cloning of hpt gene:

Serratia marcescens YT101 (FERM BP-1291) was inoculated on an LB medium and cultured at 30°C for 20 hours. Chromosomal DNA was purified from the thus cultured cells according to the well known procedure [H. Sato & K.I. Miura: Biochim, Biophys, Acta, 72, 619 (1963)]. As a vector, pUC19 purified from Escherichia coli JM109 strain [Y. Yanish-Perron et al: Gene, 33, 103 (1985)] by the well known procedure [T. Maniatis et al.: Molecular cloning, Cold Spring Harbor Lab. 1982)] was used. The JM109 strain bearing pUC19 is hereinafter referred to as "AI623 strain". The LB medium was used hereafter for culturing and preservation of microorganisms unless otherwise specified.

Then, 5 µg of purified chromosome of Serratia marcescens YT101 strain was dissolved in 40 µℓ of a buffer solution comprising 100 mM tris-hydrochloride buffer solution (pH7.5), 50 mM NaCℓ, 6 mM MgCl$_2$, 1 mM dithiothreitol and 100 µg/mℓ bovine serum albumin (hereinafter referred to as "EcoRI buffer solution"), and the resulting solution was admixed with 20 units of restriction enzyme EcoRI (made by Takara Shuzo K.K.; as restriction enzymes, those made by Takara Shuzo K.K. were used hereinafter unless otherwise specified) and incubated at 37°C for 2 hours for digestion. Then, the reaction was discontinued by a heat treatment at 65°C for 10 minutes.

The digest was admixed with 140 µℓ of distilled water and 20 µℓ of 3M sodium acetate (pH5.6), then admixed with 2-fold volume of ice-cold ethanol and allowed to stand at -80°C for 20 minutes. After centrifugation, the supernatant was removed to recover precipitates of DNA (the procedure is hereinafter referred to as "ethanol precipitation procedure"). The precipitates were admixed with 20 µℓ of distilled water to dissolve the precipitates and a concentrate of chromosomal DNA fragments was obtained.

Separately, 2 µg of pUC19 plasmid DNA was dissolved in 40 µℓ of EcoRI buffer solution, and the solution was admixed with 10 units of EcoRI and incubated for digestion at 37°C for 2 hours. Then, the reaction was discontinued by a heat treatment at 65°C for 10 minutes. Concentration of plasmid DNA fragments was carried out according to the above ethanol precipitation procedure, and the resulting precipitates were dissolved in 15 µℓ of distilled water.

The thus obtained EcoRI-digested fragments of chromosomal DNA of Serratia marcescens and EcoRI-digested fragments of pUC19 were dissolved in 50 µℓ of a buffer solution comprising 66 mM tris-hydrochloride buffer solution (pH 7.6), 6.6 mM MgCl$_2$, 10 mM dithiothreitol and 1 mM ATP, and then the resulting solution was admixed with 2 units of T4DNA ligase (made by Takara Shuzo K.K.) and incubated at 12°C for 18 hours.

The Escherichia coli AI675 strain prepared in the foregoing item (1) was transformed according to the well known procedure [T. Maniatis et al: Molecular cloning, Cold Spring Harbor Lab. (1982)] with the thus obtained recombinant plasmids and transformants having an ampicillin resistance and in which a requirement for Hyp or inosine was complemented, were obtained by selecting strains growable on an LB plate medium containing 75 µg/mℓ ampicillin and containing neither Hyp nor inosine. Plasmid DNA was purified from the thus obtained strains in the same manner as the purification of pUC19 plasmid DNA, and then the DNA was digested by EcoRI to make structural analysis of the plasmid. It was found to be a recombinant plasmid having about 15 kilobases (which kilobase is hereinafter to as "kb") of the DNA fragment originating from Serratia marcescens inserted at the EcoRI-cleaved site of pUC19, and named pAI63.

Escherichia coli AI675 strain was transformed with pAI63 and the growth of the thus transformant on plate media was investigated. The growth was observed on an M9Ap plate medium (containing 1g of NH$_4$Cl, 14.7g of Na$_2$HPO$_4 \cdot$12H$_2$O, 3g of KH$_2$PO$_4$, 5g of NaCl, 0.25g of MgSO$_4 \cdot$7H$_2$O, 15 mg of CaCl$_2 \cdot$2H$_2$O, 2g of glucose, 1 mg of vitamin B$_1$, 0.1g of arginine, 0.1g of proline, and 75 mg of ampicillin, dissolved in 1 ℓ of water and admixed with 1.5% agar), further containing 1 mM each of Hyp, inosine and guanine or guanosine, whereas no growth was observed on the M9Ap plate medium containing 1 mM xanthine. The results suggest that since the pAI63-carrying strains do not utilize xanthine as a substrate, the hpt gene originating from Serratia marcescens exists on pAI63. The structure of the thus obtained pAI63 was analyzed by digestion with various restriction enzymes and agarose gel electrophoresis. As a result, it was found that pAI63 had a structure as shown in Fig. 1.

By transforming Escherichia coli JM109 strain with pAI63, Escherichia coli AI690 carrying pAI63 was obtained. The thus obtained strain was deposited as FERM BP-1545 with the Fermentation Research Institute (FRI), Agency of Industrial Science and Technology on November 2, 1987.

(3)  Determination of HPRTase activity:

HPRTase activity was determined in the following manner:

Each of seed cultures of Escherichia coli AI675, AI623 and AI690 was inoculated on LB liquid media separately and subjected to shaking culture at 30°C for 20 hours. Then, the cells were centrifuged from the medium. Then, the separated cells were suspended in an appropriate amount of 10 mM phosphate buffer solution (pH7.0) separately and subjected to an ultrasonic treatment. Then, the disrupted cells were removed therefrom by centrifugation and the resulting supernatant fractions were used as crude enzyme solutions for the activity determination.

The thus obtained crude enzyme solutions were added to the reaction mixture comprising 100 mM tris-hydrochloride buffer solution (pH 7.0), 10 mM $MgCl_2$, 20 mM sodium fluoride, 5 mM PRPP and 2.5 mM Hyp, and the mixtures were allowed to stand at 37°C to conduct reaction to form IMP from Hyp. The amount of IMP formed by the reaction was quantitatively determined by sampling the reaction mixture with the elapse of time, boiling the mixture at 100°C for two minutes, centrifuging, diluting the supernatant fractions and analyzing by a high-performance liquid chromatography, measuring the absorbancy at 254 nm.

The activity was expressed in terms of an activity to form 1 nM IMP at 37°C for one minute as one unit, measured with a high-performance liquid chromatograph (TRIROTOR, type V, made by Nihon Bunko K.K.). The results are shown in Table 1.

Example 2

Construction of recombinant plasmid comprising a DNA fragment containing a GPRTase gene originating from Serratia marcescens:

(1) Shot gun cloning of GPRTase gene:

GPRTase also has an HPTase activity and thus transformants showing an HPTase activity are obtained by carrying out shut gun cloning in the same manner as the HPTase gene was obtained, and strains carrying a recombinant plasmid containing a GPRTase gene (hereinafter referred to as "gpt gene") can be obtained by selecting strains showing a GPRTase activity from the transformants.

Serratia marcescens YT101 (FERM BP-1291) was cultured in the same manner as in Example 1 and chromosomal DNA was separated from the thus cultured cells and purified. As a vector, pUC19 was used as in Example 1.

5 µg of purified chromosomal DNA of Serratia marcescens YT 101 strains was dissolved in 40 µℓ of EcoRI buffer solution, and the thus obtained solution was admixed with 20 units of restriction enzyme HindIII and incubated for digestion at 37°C for 2 hours. The reaction was discontinued by a heat treatment at 65°C for 10 minutes.

Precipitates were obtained from the product of digestion according to the ethanol precipitation procedure and then admixed with 20 µℓ of distilled water to dissolved the precipitates. Thus, a concentrate of chromosomal DNA fragments was obtained.

Separately, 2 μg of pUC19 plasmid DNA was dissolved in 40 μℓ of EcoRI buffer solution, and the resulting solution was admixed with 10 untis of HindIII and incubated for a disgetion at 37°C for 2 hours. The reaction was discontinued by a heat treatment at 65°C for 10 minutes. Concentration of plasmid fragments was carried out according to the ethanol precipitation procedure, and the precipitates were admixed with 15 μℓ of distilled water to dissolve the precipitates.

The thus obtained chromosomal DNA fragments of Serratia marcescens and HindIII-digested fragments of pUC19 were treated in the same manner as in Example 1 (2) to obtain recombinant plasmids. Escherichia coli AI675 strain was transformed with the thus obtained recombinant mixture in the same manner as in Example 1 and transformed strains having a resitance to ampicillin (75 μg/mℓ) and in which a requirement for Hyp or inosine was complemented, were selected. A plasmid was separated from the thus obtained transformants and purified in the same manner as the DNA of pUC19 was prepared, and the structural analysis of the plasmid was carried out by digesting the DNA with HindIII. It was found to be a recombinant plasmid having about 20 kb of DNA fragment originating from Serratia marcescens inserted at the HindIII-cleaved site of pUC19 and named pAI64.

Escherichia coli AI675 strain was transformed with pAI64 and growth of the thus obtained transformant was investigated on a plate medium. The transformant grew on an M9Ap plate medium containing 1 mM each of Hyp, inosine, guanine and guanosine and also on the plate medium containing 1 mM xanthine. The results suggest that, since the pAI64-carrying strain utilizes xanthine as a substrate, the gpt gene originating from Serratia marcescens exists on pAI64.

By transforming Escherichia coli JM109 strain with pAI64, Escherichia coli AI712 carrying pAI64 was obtained. The strian was deposited as FERM BP-1546 with the FRI on November 2, 1987.

    (2)   Determination of GPRTase activity:

    GPRTase activity was determined in the following manner.

Culturing of <u>Escherichia</u> <u>coli</u> and preparation of crude enzyme solution, to be used in the activity determination test were carried out in the same manner as in Example 1 (3).

The crude enzyme solution was added to a reaction mixture comprising 100 mM tris-hydrochloride buffer solution (pH 7.0), 10 mM $MgCl_2$, 20 mM sodium fluoride, 5 mM PRPP and 2.5 mM guanine, and the mixture was allowed to stand at 37°C conduct the reaction to form GMP from guanine.

The amount of GMP formed by the reaction was quantitatively determined by sampling the reaction mixture with the elapse of time, boiling the mixture at 100°C for 2 minutes, centrifuging the mixture, diluting the supernantant fraction and analyzing by high-performance liquid chromatography measuring the absorbancy at 254 nm. The activity was expressed in terms of the activity to form 1 nM GMP at 37°C for one minute as one unit, measured by a high-performance liquid chromatography (TRIROTOR, type V, made by Nihon Bunko K.K.)

Table 1 shows the activities of HPRTase and GPRTase.

## Table 1

### Expression of HPRTase and GPRTase genes of *Serratia marcescens* in *Escherichia coli*

| Host strain (*Escherichia coli*) | HPRTase Specific activity (Unit/μg protein) | GPRTase Specific activity (Unit/μg protein) |
| --- | --- | --- |
| AI675 | 0 | 0 |
| AI623 (JM109/pUC19) | 0.034 | 0.026 |
| AI690 (JM109/pAI63) | 1.130 | 0.132 |
| AI712 (JM109/pAI64) | 0.096 | 0.134 |

Example 3

Construction of plasmid capable of efficiently expressing GPRTase gene originating from *Escherichia coli*:

(1) Subcloning of GPRTase gene (gpt) of *Escherichia coli* into pUC19 plasmid:

*Escherichia coli* JA200 strain carrying hybrid plasmid pLC44-11 [Cell 9, 91(1976)] comprising a gpt gene originating from chromosomal DNA of *Escherichia coli*, and ColE1 was inoculated on an LB medium and cultured at 30°C for 18 hours. Plasmid pLC44-11 was purified from the thus cultured cells according to the well known procedure [T. Maniatis et al: Molecular cloning, Cold Spring Harbor Lab. (1982)]. pUC19 for use as a vector was purified from *Escherichia coli* JM109 strain according to the similar procedure. It was assumed that pLC44-11 had a size of about 30 kb and the gpt gene existed on about 1 kb of BglI-HincII fragment [R.C. Mulligan and P. Berg: Science 209, 1422 (1980)].

Then, 5 μg of the thus prepared pLC44-11 plasmid DNA was dissolved in 50 μℓ of a buffer solution comprising 10 mM tris-hydrochloride buffer solution (pH 7.5), 50 mM NaCl, 6 mM MgCl$_2$, 1 mM dithiothreitol and 100 μg/mℓ bovine serum albumin, and the thus obtained solution was admixed with 20 units of restriction enzyme BglI and incubated for digestion at 37°C for one hour. Then, the reaction solution was admixed with 20 units of HincII and incubated for digestion at 37°C for one hour. The reaction was discontinued by a heat treatment at 65°C for 10 minutes. The digested product was subjected to a low melting point agarose gel electrophoresis [Analytical Biochemistry, 98, 305 (1979)] to isolate about 1 kb of plasmid DNA fragments.

The thus obtained DNA fragments were dissolved in 40 μℓ of a reaction solution comprising 50 mM tris-hydrochloride buffer solution (pH7.6), 7 mM MgCl$_2$, 6 mM 2-mercaptoethanol, 0.25 mM dATP, 0.25 mM dGTP, 0.25 mM dTTP and 0.25 mM dCTP, and the thus obtained solution was successively admixed with 6 units of Escherichia coli DNA polymerase I Klenow fragment (1 μℓ, made by New England Biolabs) and incubated at 15°C for 2 hours to change the 3'-extruded terminal to a blunt terminal by virtue of BalI. Then, the DNA fragments were isolated by phenol-chloroform extraction and ethanol precipitation.

Separately, 2 μg of pUC19 plasmid DNA was dissolved in 20 μℓ of a buffer solution comprising 10 mM tris-hydrochloride buffer solution (pH7.5), 6 mM MgCl$_2$, 1 mM dithiothreitol and 100 μg/mℓ bovine serum albumin, and the thus obtained solution was admixed with 5 units of SmaI and incubated for digestion at 37°C for 2 hours, and 2.7 kb of plasmid DNA fragments was isolated.

About 0.2 μg of the thus obtained DNA fragments originating from pLC44-11 and about 0.5 μg of the thus obtained SmaI-cleaved DNA fragments originating from pUC19 were dissolved in 40 μℓ of a buffer solution comprising 20 mM tris-hydrochloride buffer solution (pH7.6), 10 mM MgCl$_2$, 10 mM dithiothreitol and 0.5 mM ATP, and the thus obtained solution was admixed with 2 units of T4DNA ligase and incubated at 12°C for 18 hours.

Escherichia coli AI675 strain was transformed with the thus obtained various recombinant plasmids according to the well known procedure and transformants having a resistance to ampicillin (75 µg/mℓ) in which a requirement for guanine was complemented, were selected. A plasmid was isolated from the thus obtained transformants in the same manner as the DNA of pUC19 was prepared, and the structure of the plasmid was analyzed by digesting the DNA with restriction enzymes such as EcoRI, HindIII, etc. It was found to be a recombinant plasmid having a DNA fragment containing about 1 kb of gpt gene inserted in pUC19, and named pGPT1. By transforming Escherichia coli JM109 strains with pGPT1, Escherichia coli AI737 carrying pGPT1 was obtained. The strain was deposited as FERM BP-1547 with the FRI on November 2, 1987.

　　(2)　Expression of HPRTase and GPRTase of Escherichia coli overexpressing the gpt gene of Escherichia coli:

Activities of HPRTase and GPRTase of AI675, AI623 and AI737 strains were determined in the same manner as in Examples 1 and 2, and the results are shown in Table 2.

Table 2

| Host strain (Escherichia coli) | HPRTase Specific activity (Unit/µg protein) | GPRTase Specific activity (Unit/µg protein) |
|---|---|---|
| AI675 | 0 | 0 |
| AI623 (JM109/pUC19) | 0.039 | 0.026 |
| AI737 (JM109/pGPT1) | 0.098 | 0.314 |

Example 4

A loopful of Brevibacterium ammoniagenes ATCC6872 was inoculated in a 70 mℓ-large test tube containing 10 mℓ of a seed culture comprising 1% polypeptone, 0.5% meat extract, 0.5% yeast extract and 0.25% sodium chloride (pH 7.2) and subjected to reciprocal shaking culture at 30°C for 24 hours.

A medium comprising 15% glucose, 0.01% casein hydrolyzate, 0.7% yeast extract, 1.0% ammonium sulfate, 0.3% $KH_2PO_4$, 0.3% $K_2HPO_4$, 0.5% $MgSO_4 \cdot 7H_2O$ and 10 µg/l biotin was adjusted to pH 7.2, distributed by 20 mℓ into 300 mℓ-Erlenmeyer flasks provided with buffles, and sterilized at 120°C for 20 minutes. 2 mℓ of the seed culture was inoculated into the thus obtained medium. Rotating shaking culture was carried out at 30°C for 76 hours while keeping pH around neutral by adding urea thereto, when required. After the end of culturing, the cells were recovered by centrifugation and preserved by freezing at -20°C.

10 mℓ of an LB liquid medium (pH7.2) was put into a large-scaled test tube, and sterilized.

One loopful of <u>Escherichia coli</u> ATCC10798 strain was inoculated into the medium and subjected to reciprocal shaking culture at 30°C for 20 hours. Then, 10 mℓ of the culture was inoculated into a 400 mℓ of an LB liquid medium in a 2ℓ-Erlenmeyer flask and subjected to rotating shaking culture at 37°C for 17 hours.

The cells were recovered by centrifugation and preserved by freezing at -20°C.

The frozen cells of <u>Escherichia coli</u> ATCC10798 as a converting microorganism and the frozen cells of <u>Brevibacterium ammoniagenes</u> ATCC6872 as a microorganism having an activity to form PRPP were suspended in water to make a final cell concentration of the former 50 mg/mℓ as wet cell weight and that of the latter 200 mg/mℓ as wet cell weight. The resulting suspension of the mixed cells was admixed with 100 mM Hyp (made by Kojin K.K.), 80 mg/mℓ glucose, 5 mg/mℓ sodium phytate (pH 7.0), 20 mg/mℓ $KH_2PO_4$, and 5 mg/mℓ $MgSO_4 \cdot 7H_2O$, further admixed with 4 mg/mℓ Nymeen S-215 and 10 µℓ/mℓ xylene, and distributed by 20 mℓ into 200 mℓ-beakers. The mixture was stirred with a magnetic stirrer at 900 rpm and kept at 32°C for 24 hours while adjusting the pH to about 7.4 with NaOH to conduct the reaction to phosphoribosylate Hyp to IMP. As a result, 4.02 mM IMP (corresponding to IMP·Na$_2$·7H$_2$O, the same shall apply hereinafter) was formed and accumulated. In case of adding neither Nymeen S-215 nor xylene, only 0.4 mM IMP was formed. Furthermore, in case of adding no cells of Brevibacterium ammoniagenes ATCC6872, the amount of accumulated IMP was less than 0.3 mM.

Example 5

Escherichia coli AI690 strain carrying pAI63 obtained in Example 1 was cultured under the same conditions as for culturing Escherichia coli ATCC10798 in Example 4 and centrifuged. Then, the recovered cells were preserved by freezing at -20°C. The reaction to phosphoribosylate Hyp to IMP was carried out in the same manner as in Example 4, except that the thus obtained frozen cells were used in place of Escherichia coli ATCC10798. It was found that 18.7 mM IMP was formed and accumulated in the reaction solution.

Example 6

Escherichia coli AI712 strain carrying pAI64 obtained in Example 2 was cultured under the same conditions as for culturing Escherichia coli ATCC10798 in Example 4 and centrifuged. Then, the recovered cells were preserved by freezing at -20°C. The reaction to phosphoribosylate Hyp to IMP was carried out in the same manner as in Example 4 except that the thus obtained frozen cells were used in place of Escherichia coli ATCC10798, and it was found that 7.3 mM IMP was formed and accumulated in the reaction solution.

Example 7

Escherichia coli AI737 strain carrying pGPT1

obtained in Example 3 was cultured under the same conditions as for culturing Escherichia coli ATCC10798 in Example 4 and centrifuged. Then, the recovered cells were preserved by freezing at -20°C. The reaction to phosphoribosylate Hyp to IMP was carried out in the same manner as in Example 4 except that the thus obtained frozen cells were used in place of Escherichia coli ATCC10798, and it was found that 6.9 mM IMP was formed and accumulated in the reation solution.

Example 8

Escherichia coli AI690 strain carrying pAI63, obtained in Example 1 was cultured under the same conditions as for culturing Escherichia coli ATCC10798 in Example 4 and centrifuged. Then, the recovered cells were preserved by freezing at -20°C.

Brevibacterium ammoniagenes ATCC21480 was cultured under the same conditions as for culturing Brevibacterium ammoniagenes ATCC6872 in Example 4 and centrifuged. Then, the recovered cells were preserved by freezing at -20°C.

The frozen cells of Escherichia coli AI690 strain and the frozen cells of Brevibacterium ammoniagenes ATCC21480 were suspended in water to make a final cell concentration of the former 50 mg/ml as the wet cell weight and that of the latter 200 mg/ml as the wet cell weight. The thus obtained suspension of mixed cells was admixed with 110 mM Hyp, 15 mg/ml ribose, 80 mg/ml glucose, 5 mg/ml sodium phytate (pH7.0), 20 mg/ml $KH_2PO_4$ and 5 mg/ml $MgSO_4 \cdot 7H_2O$, further with 4 mg/ml Nymeen S-215 and 10 µl/ml xylene, and then the mixture was distributed by 20 ml in 200 ml-beakers. The mixture was stirred with a magnetic stirrer at 900 rpm and kept at 32°C for 24 hours, while adjusting the pH to about 7.4 with NaOH to conduct the reaction to phosphoribosylate Hyp to IMP. As a result, 86 mM IMP was formed and accumulated in the reaction solution.

## Example 9

The reaction to phosphoribosylate Hyp to IMP was carried out in the same manner as in Example 8 except that the individual strains shown in Table 3 were used as converting microorganisms in place of Escherichia coli ATCC10798 strain. The resutls are shown in Table 3.

### Table 3

| Strain | | Amount of IMP formed (mM) |
|---|---|---|
| Escherichia coli | ATCC11303 | 5.57 |
| Bacillus subtilis | ATCC14617 | 3.44 |
| Flavobacterium devorans | ATCC10829 | 4.09 |
| Serratia marcescens | FERM BP-1291 | 5.51 |
| Salmonella typhimurium | ATCC19585 | 3.80 |
| Lactobacillus casei | ATCC7469 | 3.57 |
| Corynebacterium glutamicum | ATCC13032 | 4.47 |
| Brevibacterium ammoniagenes | ATCC6872 | 4.83 |

## Example 10

The reaction to phosphoribosylate Hyp to IMP was carried out in the same manner as in Example 4 except that various strains shown in table 4 were used as microorganisms having an activity to form PRPP in place of Brevibacterium ammoniagenes ATCC6872 strain. The results are shown in Table 4.

Table 4

| Strain | | Amount of IMP formed (mM) |
|---|---|---|
| Corynebacterium glutamicum | ATCC13032 | 3.31 |
| Bacillus subtilis | ATCC14618 | 3.22 |
| Escherichia coli | ATCC33525 | 3.85 |
| Staphylococcus aureus | ATCC4012 | 2.46 |
| Saccharomyces sake | ATCC20018 | 2.23 |
| Candida zeylanoides | ATCC20356 | 1.98 |
| Torulopsis psychrophila | ATCC22163 | 2.37 |

Claims:

1. A process for producing 5'-inosinic acid, which comprises conducting the reaction to form 5'-inosinic acid in the presence of a microorganism having an activity to form 5-phosphoribosyl-1-pyrophosphate or its precursor, a microorganism having an activity to form inosinic acid from hypoxanthine or its precursor and 5-phosphoribosyl-1-pyrophosphate or its precursor, or a treatment product of their cultures or cells, hypoxanthine or its precursor or a material containing hypoxanthine and its precursor, a carbon source and a phosphate donor, thereby forming and accumulating 5'-inosinic acid in the reaction solution, and recovering 5'-inosinic acid therefrom.

2. The process according to Claim 1, wherein as the microorganism having an activity to form 5'-inosinic acid from hypoxanthine and 5-phophoribosyl-1-pyrophosphate or its precursor, a microorganism carrying a recombinant DNA comprising a DNA fragment containing a gene coding for an enzyme having an activity to phosphoribosylate hypoxanthine and a vector DNA, is used.

3. The process according to Claim 2, wherein the microorganism is a microorganism belonging to the genus Escherichia coli and carrying a recombinant DNA comprising a DNA fragment containing a gene coding for an enzyme having an activity to phosphoribosylate hypoxanthine and a vector DNA.

4. The process according to any one of Claims 2 and 3, wherein as the DNA fragment comprising a gene coding for an enzyme having an activity for phosphoribosylating hypoxanthine, a DNA fragment originating from a chromosomal DNA of a microorganism belonging to Escherichia coli, Bacillus subtilis, Flavobacterium devorans, Serratia marcescens, Salmonella typhimurium, Lactobacillus casei, Brevibacterium ammoniagenes or Corynebacterium glutamicum is used.

5.     The process according to any one of Claims 2 to 4, wherein the gene coding for an enzyme having an activity to phosphoribosylate hypoxanthine is a gene coding for hypoxanthine phosphoribosyl transferase originating from Serratia marcescens, or a gene coding for guanine-xanthine phosphoribosyl transferase originating from Escherichia coli or Serratia marcescens.

6.     The process according to any one of Claims 1 to 5, wherein the treatment of the cells is carried out by contacting the cells with an organic solvent and/or a surfactant in advance or adding the organic solvent and/or the surfactant to the reaction solution.

7.     A recombinant DNA comprising a DNA fragment containing a gene coding for hypoxanthine phosphoribosyl transferase originating from Serratia marcescens or a DNA fragment containing a gene coding for guanine-xanthine phosphoribosyl transferase originating from Escherichia coli or Serratia marcescens, and a vector DNA.

8.     A microorganism belonging to the genus Escherichia and carrying a recombinant DNA comprising a DNA fragment containing a gene coding for hypoxanthine phosphoribosyl transferase originating from Serratia marcescence or a DNA fragment containing a gene coding for guanine-xanthine phosphoribosyl transferase originating from Escherichia coli or Serratia marcescens, and a vector DNA.

9.     The microorganism according to Claim 8, wherein the microorganism is a microorganism selected from Escherichia coli AI690 (FERM BP-1545), Escherichia coli AI712 (FERM BP-1546) and Escherichia coli AI737 (FERM BP-1547).

EP 0 406 436 A1

Fig. 1

E    Sm  Sc    Sc Bg K            Xh      M K E        E K Sm H  Sc    E

pAI63 (17.4 kb)                                        pUC19

0                    5                   10                   15          (kb)

# INTERNATIONAL SEARCH REPORT

International Application No   PCT/JP89/01187

## I. CLASSIFICATION OF SUBJECT MATTER (if several classification symbols apply, indicate all) ⁶

According to International Patent Classification (IPC) or to both National Classification and IPC    Int. Cl⁵

C12P19/32, C12N15/54, 15/70, 1/21//(C12P19/32, C12R1:19)(C12N15
/54, C12R1:425)(C12N1/21, C12R1:19)(C12P19/32, C12R1:13, 1:19)

## II. FIELDS SEARCHED

### Minimum Documentation Searched ⁷

| Classification System | Classification Symbols |
|---|---|
| IPC | C12P19/32, C12N15/54, 15/70, 1/21 |

### Documentation Searched other than Minimum Documentation to the Extent that such Documents are Included in the Fields Searched ⁸

Biological Abstracts Data Base (BIOSIS)
Chemical Abstracts Data Base (CA, REGISTRY)

## III. DOCUMENTS CONSIDERED TO BE RELEVANT ⁹

| Category * | Citation of Document, ¹¹ with indication, where appropriate, of the relevant passages ¹² | Relevant to Claim No. ¹³ |
|---|---|---|
| Y | JP, B1, 48-39477 (Kyowa Hakko Kogyo Co., Ltd.), 24 November 1973 (24. 11. 73), & FR, A1, 1480009 & GB, A, 1139097 | 1, 6 |
| Y | JP, B1, 44-24309 (Kyowa Hakko Kogyo Co., Ltd.), 15 October 1969 (15. 10. 69) | 1, 6 |
| Y | JP, B1, 41-16560 (Kyowa Hakko Kogyo Co., Ltd.), 19 September 1966 (19. 09. 66) | 1, 6 |
| Y | JP, B1, 45-11556 (Kyowa Hakko Kogyo Co., Ltd.), 25 April 1970 (25. 04. 70), & FR, A1, 1473629 & US, A, 3674641 & GB, A, 1092597 & DE, A1, 1517820 | 1, 6 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| February 14, 1990 (14. 02. 90) | February 26, 1990 (26. 02. 90) |

| International Searching Authority | Signature of Authorized Officer |
|---|---|
| Japanese Patent Office | |

Form PCT ISA:210 (second sheet) (January 1985)